(19) Europäisches Patentamt
European Patent Office
Office européen des brevets

(11) **EP 4 479 104 B1**

(12) **EUROPEAN PATENT SPECIFICATION**

(45) Date of publication and mention
of the grant of the patent:
**08.04.2026 Bulletin 2026/15**

(51) International Patent Classification (IPC):
*A61L 27/34* (2006.01)      *A61L 27/44* (2006.01)
*A61L 27/58* (2006.01)      *C03C 4/00* (2006.01)
*C03C 3/097* (2006.01)      *C03C 25/40* (2006.01)

(21) Application number: **23704810.3**

(52) Cooperative Patent Classification (CPC):
**A61L 27/34; A61L 27/446; A61L 27/58;
C03C 3/097; C03C 4/0014; C03C 25/40**

(22) Date of filing: **16.02.2023**

(86) International application number:
**PCT/EP2023/053956**

(87) International publication number:
**WO 2023/156558 (24.08.2023 Gazette 2023/34)**

(54) **RESORBABLE GLASS FIBER COATED WITH A SIZING AND METHOD OF PREPARING SUCH**

MIT EINER SCHLICHTE BESCHICHTETE RESORBIERBARE GLASFASER UND VERFAHREN ZUR HERSTELLUNG DERSELBEN

FIBRE DE VERRE RÉSORBABLE REVÊTUE D'UN ENSIMAGE ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **16.02.2022 US 202263310831 P
25.02.2022 EP 22158900**

(43) Date of publication of application:
**25.12.2024 Bulletin 2024/52**

(73) Proprietor: **Purac Biochem B.V.
4206 AC Gorinchem (NL)**

(72) Inventors:
• **CANADELL-AYATS, Judit
4206 AC Gorinchem (NL)**
• **HASAN, Muhammed Sami
Tucker, GA 30084 (US)**
• **LIU, Jenq
Tucker, GA 30084 (US)**
• **DE VOS, Siebe Cornelis
4206 AC Gorinchem (NL)**
• **PENISTON, Shawn James
Tucker, GA 30084 (US)**

(74) Representative: **De Vries & Metman
Overschiestraat 180
1062 XK Amsterdam (NL)**

(56) References cited:
**CN-A- 106 149 127      US-A1- 2012 040 015**

## Description

### Field of the invention

The invention relates to resorbable and biocompatible glass fibers which are coated with a sizing. The invention further relates to a method for coating a resorbable, biocompatible glass fiber, and to a kit of parts suitable for use therein.

### Background

Glass fibers are high-performing materials that are used in many technological areas where excellent mechanical properties combined with relatively low weight is required, such as in aerospace, automotive, marine, energy, sports equipment, household items, and medical implants. They are often used in composites, where fibers, most frequently carbon or glass, are embedded in a matrix of another dissimilar material, such as polymeric materials.

Composite materials comprising glass fibers embedded in a polymeric matrix are considered to be very useful for medical applications, especially for orthopedic implants for bone and cartilage repair. Composites can be produced to be biocompatible and resorbable, meaning that they disintegrate and are resorbed in a mammalian (e.g. human) body according to a controlled rate when implanted. This allows to produce implants that do not have to be removed from the body and thus do not need a second surgery.

The performance of these composites in general depends on many factors, such as the amount, type, length, thickness and alignment of the glass fibers, the content and properties of the matrix material, and the quality of the interfacial bonding between the polymeric matrix material and the glass fibers. One way to improve the quality of the interfacial bonding between the polymeric matrix material and the glass fibers is the application of a sizing.

A glass fiber sizing contains components with different functions. Primary components are a film former (also called a compatibilizer) and a coupling agent.

The film former forms a thin film on the glass fibers and serves several functions. In particular, it improves the processability of the glass fiber, and the performance of the final product. Among others, it protects and lubricates the fibers and holds them together during composite manufacturing. In addition, and perhaps counter-intuitively, it also helps to spread the glass fibers during the wet impregnation step of composite fabrication. If the glass fiber is to be used in a composite in combination with a polymer matrix, the film formers in the sizing are usually chemically similar to the polymer matrix of the composite. This improves the adherence of the polymer matrix to the glass fibers. It also ensures good wetting of the sized glass fibers by the matrix polymer by lowering the surface energy. In this context it should be noted that the surface of glass fibers generally is hydrophilic in nature, while the polymer matrix often has a hydrophobic character.

In a sizing compatible with a thermoplastic polymer matrix, the film former should ideally be covalently bonded, i.e. grafted, to the glass fiber surface via a coupling agent such as a silane. A grafted film former that is chemically similar to the hydrophobic matrix polymer can provide a strong interface between matrix polymer and glass fiber through adhesion and chain-entanglements. This kind of entanglement can help to efficiently transfer load from matrix polymer to reinforcing fibers hence providing high initial mechanical strength as well as strength retention in-vitro or in-vivo.

For a sizing to be effective, it is of course necessary that it is maintained on the glass during processing. From J.L. Thomason, Glass fibre sizing: A review, Composites Part A 127 (2019) 105619, it is known that up to 80% of the size on glass fibers can be removed by extraction with acetone. This indicates that a portion of the film former, as well as perhaps other components, may not be chemically bonded to the glass fibers but instead are physiosorbed onto the surface. A high level of physiosorbed sizing limits the strength of the obtained composite and will unfavorably affect the strength retention of a glass reinforced composite, implant, or device in vivo.

CN 106149127 A1 discloses a biocompatible fibre yarn, comprising biological absorbable and bioactive glass fibre, a polylactide compatibilizer (PLDLA7030 polymer) and a coupling agent which forms a polar covalent bond. The coupling agent is an organosilane (alkoxyl silane), an organic titanate (alkane epoxide titanate esters or chelating titanate) or an organozirconate (alcoxyl base zirconate or alkylzirconate).

Thus, a need for stronger, biocompatible and resorbable composites remains, as well as a need for simpler methods of making such products. There is also a need for better sizing materials and methods of applying such sizing materials to resorbable and biocompatible glass fibers.

### Summary of the invention

The invention relates to a resorbable, biocompatible glass fiber, coated with a sizing wherein the sizing comprises a thermoplastic, resorbable and biocompatible compatibilizer that is covalently bonded to the glass fiber through a coupling agent having at least one silane moiety, wherein the glass wherein the glass fiber has an apparent interfacial shear strength with a reference matrix polymer as determined in the single fiber pullout test which is at least 10% higher than the apparent interfacial shear of a bare glass fiber with the same reference matrix polymer, wherein the thermoplastic, resorbable and biocompatible compatibilizer that is covalently bonded to the glass fiber through a coupling agent having at least one silane moiety is the reaction product of a coupling agent having at least one epoxy moiety and at least one silanol group and the product of the reaction of a compatibilizer which is a thermoplastic, resorbable and biocompatible polymer comprising carboxylic acid end-groups with a volatile tertiary amine in an aqueous liquid medium.

The invention further relates to a method for providing a resorbable and biocompatible glass fiber with a sizing, comprising the steps of:

a) coating a glass fiber with a sizing composition by contacting the glass fiber with

- a coupling agent with at least one epoxy moiety and at least one silanol group in a liquid medium and
- the product of the reaction of a compatibilizer with a volatile tertiary amine in an aqueous liquid medium, in which the compatibilizer is a thermoplastic, resorbable and biocompatible polymer comprising carboxylic acid end-groups,

b) subjecting the coated glass fiber to a drying step,
c) subjecting the dried coated glass fiber to a curing step at elevated temperature,
d) subjecting the cured coated glass fiber to a step to evaporate the volatile tertiary amine.

The sizing composition used in the method according to the invention is preferably waterbased. It contains the reaction product of a thermoplastic, resorbable and biocompatible polymer comprising carboxylic acid end-groups, i.e., the compatibilizer, with a volatile tertiary amine. Not wishing to be bound by theory, it is believed that this product contains ionic clusters (e.g., in the form of an alkylammonium carboxylate salt) which act as an ionic emulsifier, which preferentially occupies the interface between the dispersed compatibilizer and the surrounding water, leading to a micro- or even a nanodispersion. The volatile tertiary amine thus acts to facilitate the dispersion of the compatibilizer in the water. Additionally, in some embodiments, it also catalyzes the reaction of the compatibilizer with the silane coupling agent.

When the dispersed compatibilizer and the silane coupling agent have been applied onto the glass fibers in a liquid medium, a drying step is carried out. In the drying step the liquid medium is removed. Further, the silanol groups of the coupling agent react with the glass fiber, and the resulting reaction water is also removed. During the subsequent curing of the glass fibers the alkylammonium carboxylate salt decomposes, regenerating the carboxylic acid functionality, which then reacts with the epoxy moiety of the coupling agent. Depending on the selection of the volatile tertiary amine, this reaction may be catalyzed by the tertiary amine which is also released in the decomposition of the alkylammonium carboxylate salt. The tertiary amine can be removed, e.g., through evaporation through the application of vacuum or an inert gas stream. In one embodiment the process is controlled in a way that sufficient tertiary amine is present in the system to catalyze the reaction between the carboxylic acid groups and the epoxy groups from the silane coupling agent.

As discussed above, the use of a volatile tertiary amine in the compatibilizer is designed to function as a temporal reactive surfactant facilitating its dispersibility in (aqueous) emulsions and suspensions without the need for additional and possibly detrimental, surfactants. Furthermore, The compatibilizer is designed to react with the epoxy groups of the silane coupling agent leading to high grafting efficiencies on the glass, which facilitates in turn adhesion and physical interactions with the matrix polymer.

The invention further relates to a kit of parts for performing the method of the invention to obtain the coated glass fibers of the invention. The invention also relates to a composite comprising the sized glass fibers, and to a medical device comprising the composite.

**Detailed description**

The invention relates to resorbable and biocompatible glass fibers, coated with a sizing comprising a thermoplastic, resorbable and biocompatible compatibilizer, wherein the sizing is covalently bonded to the glass fiber through a coupling agent having at least one silane moiety. It is hereby understood that sizing or size refers to a coating on the glass fibers comprising a coupling agent that is covalently bonded to the glass and that is covalently bonded to a compatibilizer. The glass fiber has an apparent interfacial shear strength with a reference matrix polymer as determined in the single fiber pullout test which is at least 10% higher than the apparent interfacial shear of a bare glass fiber with the same reference matrix polymer, wherein the thermoplastic, resorbable and biocompatible compatibilizer that is covalently bonded to the glass fiber through a coupling agent having at least one silane moiety is the reaction product of a coupling agent having at least one epoxy moiety and at least one silanol group and the product of the reaction of a compatibilizer which is a thermoplastic, resorbable and biocompatible polymer comprising carboxylic acid end-groups with a volatile tertiary amine in an aqueous liquid medium.

A coupling agent refers to a compound that is capable of providing a chemical bond between two dissimilar materials. Typically, these are molecules having a functional group that can bind with an organic material (e.g. a polymer such as a polyester) as well as having a functional group that can bind with inorganic materials (e.g. glass, such as a glass fiber). The coupling agent of the invention comprises at least one silane moiety. It is hereby understood that silane refers to a (functional) group or to a molecule having central silicon atom and four attachments. An example of a silane is silane tetrahydride, which is a silicon atom surrounded by four hydrogen atoms. The coupling agent will be discussed in more detail below.

A compatibilizer refers to a low molecular weight polymer that is miscible with the polymer matrix of the composite in which the glass fiber is to be incorporated. The reaction product of the volatile tertiary amine and the compatibilizer in the present invention is preferably designed to function as a temporal processing aid facilitating the dispersibility of the compatibilizer in aqueous emulsions and suspensions without the need for additional surfactants. It is preferred not to have additional surfactants as these can have detrimental effects, such as leaching from the product during manufacturing or end-use. The compatibilizer is designed to react with the coupling agent such that it has a high grafting efficiency on the glass, which facilitates in turn adhesion and physical interactions, e.g. chain entanglements, with the matrix polymer of the composite in which the sized glass fibers may be used. The compatibilizer is discussed in more detail below.

Preferably the dry weight of the sizing is at least 0.1 wt.% of the coated glass fiber, more preferably at least 0.2 wt.% of the coated glass fiber, in particular 0.3 wt.% of the coated glass fiber, more preferably at least 0.4 wt.% of the coated glass fiber, more preferably at least 0.5 wt.% of the coated glass fiber and even more preferably at least 0.6 wt.% of the coated glass fiber. Preferably the dry weight of the covalently bonded sizing is at most 5 wt.%, in particular at most 3 wt.%, more in particular at most 1.2 wt.% the coated glass fiber, more preferably at most 1.0 wt.% the coated glass fiber, more preferably at most 0.8 wt.% the coated glass fiber, and even more preferably at most 0.6 wt.% of the coated glass fiber. Lower values, e.g., at most 0.3 wt.% are also possible. It is hereby understood that the coated glass fiber refers to the glass fiber including the covalently bonded sizing, i.e., including the coupling agent and the compatibilizer.

Throughout this text, the mention of glass fiber will be understood to refer to the coated glass fiber, unless it is indicated that this is not the case or it is obvious from the description that it is not the case.

A volatile tertiary amine is used in the process of the invention, and is removed through evaporation. It is preferred for the volatile tertiary amine to be substantially removed, more preferably completely. Preferably the amount of volatile tertiary amine in the coated glass fibers after drying and curing is less than 10 ppm or below the detection limit of gas chromatography-mass spectrometry (GC-MS). It is hereby understood that tertiary amine refers to a compound having a nitrogen atom, a lone electron pair, and three organic substitutes. It is possible that some volatile tertiary amine remains in the glass fiber, even after the evaporation step. Therefore, in some embodiments, the coated glass fiber of the invention contains at least 0.1 ppm of tertiary amine, in some embodiments at least 0.2 ppm, or at least 0.3 ppm. As indicated above, the amount of volatile tertiary amine in the coated glass fibers after drying and curing is preferably less than 10 ppm.

The thickness of the applied sizing is preferably at most 200 nm, more preferably at most 150 nm, more preferably at most 120 nm, more preferably at most 100 nm and even more preferably at most 75 nm. It is understood that thinner sizing layers can be beneficial especially for glass fibers with a diameter below 6 $\mu$m.

The sizing, comprising a thermoplastic, resorbable and biocompatible polyester that is covalently bonded to the glass fiber through a coupling agent having at least one silane moiety, increases the strength of the interfacial bond between the glass fiber and a polymer matrix surrounding it after incorporation into a composite material. This effect can be quantitively determined using the single fiber pull-out test. In this test method, a single sized glass fiber is embedded in a molten polymer using suitable equipment. The fiber is subsequently pulled out using suitable equipment, and the apparent interfacial shear strength is calculated. The method is discussed in more detail below. The experiment can also be performed on fibers without compatibilizer coating. By comparing the results of the fiber with and without a compatibilizer coating, a quantification of the effect of the compatibilizer coating on the debonding force from the polymer matrix can be obtained.

In the present invention, the coated glass fiber has an apparent interfacial shear strength with a reference matrix polymer as determined in the single fiber pullout test which is at least 10% higher than the apparent interfacial shear of a bare glass fiber with the same reference matrix polymer.

Within the context of this definition a reference matrix polymer is a matrix polymer which has a chemical composition which corresponds to the chemical composition of the compatibilizer. For example, if the compatibilizer is polycaprolactone, the corresponding reference matrix polymer for this definition is polycaprolactone. This parameter thus allows quantification of the quality of bonding of the sizing to the glass fiber with reference to a standard matrix material, namely a polymer with the same composition as the compatibilizer. Within the context of this definition a bare glass fiber is a coated glass fiber which has been burned in an over at 500ºC for 2 hours in the presence of oxygen (generally air). This treatment removes the compatibilizer. A comparison between the apparent interfacial shear strength values obtained for the coated glass fiber and the bare glass fiber compared to a standard matrix thus allows determination of the effect of the sizing in a reference matrix.

Preferably, the coated glass fiber has an apparent interfacial shear strength with a reference matrix polymer as determined in the single fiber pullout test which is at least 20% higher than the apparent interfacial shear of a bare glass fiber with the same reference matrix polymer, in particular at least 30%.

In one embodiment, the coated glass fiber has an apparent interfacial shear strength with a reference matrix polymer as determined in the single fiber pullout test of at least 10 MPa, in particular at least 15 MPa, more in particular at least 20 MPa. The apparent interfacial shear strength as compared to a reference matrix polymer as measured by the single fiber pull-out test preferably is at least 30 MPa, more preferably at least 40 MPa, and even more preferably at least 50 MPa.

It should be kept in mind that when the coated glass fiber is incorporated into other matrix materials, different values for the single fiber pullout test will be obtained, depending on the matrixcompatibilizer combination.

The (uncoated) glass fibers used in the present invention preferably have a diameter, determined in accordance with ASTM D 1577-01C, below 30 $\mu$m, in particular below 25 $\mu$m, more in particular below 20 $\mu$m in some embodiments below 15 $\mu$m, or even below 10 $\mu$m. Preferably, the diameter is at least 3 $\mu$m, in particular at least 5 $\mu$m, in particular at least 6 $\mu$m, or at least 8 $\mu$m. In one embodiment the glass fibers originate from a glass fiber bundle in which the coefficient of variation of the diameter of the glass fibers in the glass fiber bundle is at most 15%, in particular at most 10%. The coefficient of variation may be at most 8%, or at most 6%. It has been found that in some embodiments the coefficient of variation may be at most 3%, which is a measure of a very even diameter indeed. The coefficient of variation is determined as follows: For 30 individual glass fibers in a glass fiber bundle, the diameter is determined. The average diameter and the standard deviation are calculated. The coefficient of variation is the standard deviation divided by the average diameter, expressed in %.

Glass fiber bundles that may be used in the present invention preferably have at least 50 fibers, preferably at least 100, more preferably at least 150, and even more preferably at least 200 fibers. As a maximum, a value of 5000 fibers may be mentioned, more in particular 3000. Glass fiber bundles that may be used in the present invention preferably have a linear density of between 20 and 1300 tex, in particular between 50 and 500 tex for the total bundle, preferably between 70 and 300 tex, as measured according to ASTM D1577-01 A. The term linear density corresponds to the weight of a certain length of the glass fiber bundle and the unit tex corresponds to the weight in grams per 1000 meters of fiber.

Glass is always made up of different ingredients. These are often divided into three categories: 1) network formers, 2) network modifiers and 3) intermediates. Network formers, which are the major components of glass, refers to a class of ingredients that forms the highly cross-linked backbone network that gives the glass most of its properties. Network modifiers refers to a class of ingredients that can be added to tweak the physical properties of the glass to meet certain specifications. Network modifiers usually reduce glass network connectivity. Then there are also ingredients that can take part in the glass network and from there tweak the glass properties. These ingredients belong to the class of intermediates.

The glass fibers used in the present invention preferably have a composition comprising network formers and network modifiers, wherein the molar ratio between the network formers and network modifiers is between 1 and 4, preferably between 1.5 and 3.5, more preferably between 2 and 3. It has been observed that ratios according such a range, and in particular one of the preferred ranges leads to a good glass quality, which is particularly suitable for fiber formation.

In a preferred embodiment, the glass fibers are bioactive glass fibers. Bioactive glass fibers are known in the art and have been designed to elicit or modulate biological activity. Bioactive material often is surface-active material that can interact with mammalian tissue. Bioactive glass may further be designed to leach ions or other chemicals resulting in osteoconductive, osteoinductive, anti-infective and/or angiogenic benefits.

The glass fibers preferably comprise network formers which are selected from oxides of silicon, such as silica ($SiO_2$), oxides of boron, such as diboron trioxide ($B_2O_3$) and boron suboxide ($B_6O$), and oxides of phosphorous, such as phosphorous trioxide ($P_2O_3$) and phosphorus pentoxide ($P_2O_5$ or $P_4O_{10}$). The glass fibers preferably comprise network modifiers which are selected from oxides of sodium, such as $Na_2O$ or $Na_2O_2$, oxides of magnesium, such as MgO, and oxides of calcium, such as CaO. More preferably, the glass fibers comprise several network formers and several network modifiers.

The glass fibers preferably have a composition comprising

50 - 75 wt.% of $SiO_2$, in particular 55-75 wt.%, more in particular 60-75 wt.%,
0 - 15 wt.% of $B_2O_3$,
0.5 - 5 wt.% of $P_2O_5$, in particular 0.5-4 wt.%, more in particular 0.5-3 wt.%,
5 - 20 wt.% of $Na_2O$,
0 - 25 wt.% of CaO, in particular 2-25 wt.%, more in particular 5-25 wt.%.
0 - 10 wt.% of MgO,
0 - 1 wt.% of $Li_2O$,
0 - 15 wt.% of $K_2O$, in particular 0-10 wt.%, more in particular 0-4 wt.%,
0 - 4 wt.% of SrO,
0 - 5 wt.% of $Al_2O_3$, and
0 - 5 wt.% of $Fe_2O_3$
(not calculating the sizing).

In one embodiment, the glass fibers (not calculating the sizing) have a composition comprising 60 - 75 wt.% of $SiO_2$, 0 - 15 wt.% of $B_2O_3$, 0.5 - 3 wt.% of $P_2O_5$, 5 - 20 wt.% of $Na_2O$, 5 - 25 wt.% of CaO, 0 - 10 wt.% of MgO, 0 - 1 wt.% of $Li_2O$, 0 - 4 wt.% of $K_2O$, 0 - 4 wt.% of SrO, 0 - 5 wt.% of $Al_2O_3$, and 0 - 5 wt.% of $Fe_2O_3$.

In one embodiment, the glass composition comprises less than 10 wt.% of $B_2O_3$, in particular less than 5 wt.%. In one embodiment, the glass composition comprises 7-20 wt.% $Na_2O$. In one embodiment, the glass composition comprises 2-8 wt.% MgO. In one embodiment, the glass composition comprises 5-15 wt.% CaO.

The glass fibers used in the present invention preferably are radiopaque, meaning that they are not transparent to X-rays. It is understood that X-rays refers to high-energy radiation having a wavelength from about 10 pm to 10 nm. The use of radiopaque glass fibers is particularly attractive in composites for implantable medical devices because it allows monitoring of the degradation of the composite through X-ray in-vivo.

The glass fibers preferably have a tensile strength of 1000 - 3000 MPa, preferably between 1200 - 2500 MPa, more preferably 1400 - 2200 MPa, as measured by tensile testing according to DIN EN ISO 5079.

The glass fibers preferably have an elastic modulus between 20 - 100 GPa, preferably between 35 - 85 GPa, more preferably between 50 - 70 GPa as measured by tensile testing according to DIN EN ISO 5079 "Determination of breaking force and elongation at break of individual fibres" (ISO 5079:2020).

Attractive glass fibers and glass fiber bundles for use in the present invention are described in a patent application with the same applicant, inventors, and filing date as the present application, with the title "Resorbable and biocompatible glass fiber bundle having a well-defined diameter and process for making such".

The invention also relates to a composite comprising a plurality of coated glass fibers according to the invention, wherein the plurality of coated glass fibers is embedded in a resorbable polymer matrix. The resorbable polymer matrix can be any suitable resorbable polymer matrix known in the art. Preferably the resorbable polymer matrix is polylactide. Further examples will be given below.

The invention further relates to a medical device comprising the coated glass fibers or the composite of the present invention. Examples of medical devices are typically medical implants which may include bone fixation devices, intramedullary nails, joint (hip, knee, elbow) implants, spine implants, and other implants such as for fracture fixation, tendon reattachment or spinal fixation.

Specific examples of bone fixation devices are screws, plates, rods, tapes, nails, wires, pins, tapes, anchors, cables, ties, or wire ties, plate and screw systems, and external fixators.

The coated glass fibers or the composite of the present invention can also be used in tissue engineering for example in woven and non-woven fabrics and scaffolds.

The invention further relates to a method for providing a resorbable and biocompatible glass fiber with a sizing, comprising the steps of:

a) coating a glass fiber with a sizing composition by contacting the glass fiber with

- a coupling agent with at least one epoxy moiety and at least one silanol group in a liquid medium and
- the product of the reaction of a compatibilizer with a volatile tertiary amine in an aqueous liquid medium, in which the compatibilizer is a thermoplastic, resorbable and biocompatible polymer comprising carboxylic acid end-groups,

b) subjecting the coated glass fiber to a drying step,
c) subjecting the dried coated glass fiber to a curing step at elevated temperature,
d) subjecting the cured coated glass fiber to a step to evaporate the volatile tertiary amine.

In the present specification, the reaction product of the reaction of a compatibilizer with a volatile tertiary amine, in which the compatibilizer is a thermoplastic, resorbable and biocompatible polymer comprising carboxylic acid end-groups is sometimes indicated with the wording "neutralized compatibilizer". The word "neutralized" implies the presence of an ionic cluster comprising a negatively charged carboxylic acid group and a positively charged amine group.

In step a) of the method according to the invention, a glass fiber is coated with a sizing composition by contacting the glass fiber with a coupling agent in a liquid medium and the neutralized compatibilizer in a liquid medium. In one embodiment, the coupling agent and the neutralized compatibilizer are present in a single liquid medium. In that case step a) comprises coating a glass fiber with a sizing formulation comprising, in a liquid medium, the product of the reaction of a compatibilizer with a volatile tertiary amine, in which the compatibilizer is a thermoplastic, resorbable and biocompatible polymer comprising carboxylic acid end-groups, and a coupling agent with at least one epoxy moiety and at least one silanol group. In another embodiment, the coupling agent and the neutralized compatibilizer are provided in separate steps, in individual liquid media. In that case, the glass fiber is generally first contacted with a liquid medium comprising a coupling agent, followed by contacting the glass fiber with a liquid medium containing the neutralized compatibilizer. If so desired, an intermediate drying step, and optionally, an intermediate curing step may be carried out. The contacting step can be carried out as is known in the art, e.g., by passing the fibers, e.g., in a fiber bundle, through a bath of the liquid medium.

The liquid medium is an aqueous medium. In this case, the neutralized compatibilizer is present in the form of a dispersion, in particular a microdispersion or even a nanodispersion. This dispersion may be formed by providing a compatibilizer comprising an amount of a thermoplastic, resorbable and biocompatible polymer comprising carboxylic acid moieties, neutralizing at least part of the carboxylic acid moieties of the compatibilizer with a predetermined amount of a tertiary amine, and dispersing the neutralized compatibilizer in an liquid medium, in particular in an aqueous liquid medium. The compatibilizer may, e.g., be provided in the form of a melt, or it may be provided in the form of a solution in a suitable solvent.

The tertiary amine used in the method of the invention is chosen such that it is volatile. In the context of the present specification the term volatile means that the amine can be evaporated from the glass fiber provided with the sizing. Additionally, the tertiary amine used in the method is preferably chosen such that it can act as a catalyst for the reaction between the coupling agent and the compatibilizer. Thus, preferably the tertiary amine used in the method can act as a catalyst for the reaction between the at least one epoxy moiety of the coupling agent and a carboxylic acid moiety of the compatibilizer.

After the provision of the glass fiber with the sizing composition, in one or two steps, whether or not with intermediate drying and/or curing as described above, the coated glass fiber is subjected to a drying step. In the drying step the liquid medium, preferably water is removed. Further, if this has not taken place yet in a two-step process as described above, the silanol groups of the coupling agent react with the glass fiber, and the resulting reaction water is also removed. The drying step is generally carried out under relatively mild conditions. This is because it is attractive to ensure preservation of part of the tertiary amine to serve as a catalyst for the reaction between the terminal acid group of the compatibilizer and the epoxy group on the silane coupling agent. Suitable reaction conditions include a temperature in the range of 0-50 °C. Preferably the water is removed at about room temperature (i.e., 15-25 °C) under reduced pressure (i.e., lower than 100 kPa, preferably lower than 50 kPa).

The silane moiety of the coupling agent makes hydrogen bond with the -OH group present on the glass fiber surface. This bond eventually converts into a covalent bond with glass fiber after losing a water molecule in the curing step. Hence the reaction between the epoxy moiety of the coupling agent and the glass is considered a solid-state reaction resulting in grafting of the silane to the solid glass via the silanol bond.

The dried coated glass fiber is subjected to a curing step at elevated temperature. As indicated above, in the curing step, the reaction between the silane moiety of coupling agent with - OH groups on the glass fiber surface is completed to form a silanol bond and the reaction between the epoxy moiety of the coupling agent and the neutralized compatibilizer is performed. This is preferably carried out above the glass transition temperature of the compatibilizer. Hence the reaction between the epoxy moiety and the neutralized compatibilizer is preferably carried out at a temperature of at least 70 °C, more preferably at least 80°C, more preferably at least 90°C and even more preferably at least 100 °C. In order to prevent thermo-oxidative damage of the coupling agent and/or compatibilizer, it might also be preferred to limit the temperature to a maximum of 140 °C or more preferably to a maximum of 120 °C. It is hence understood that the reaction between the epoxy moiety and the neutralized compatibilizer is preferably carried out at a temperature of 80 - 140 °C, more preferably of 100 - 120 °C.

The coupling agent comprises an epoxy (i.e. glycidyl) group for binding to the carboxylic acid moieties. The coupling agent further comprises a silanol group for binding to the glass. In one embodiment, the silanol group is formed by reacting a compound with an alkoxysilane group with (a limited amount of) water to convert the alkoxysilane group to a silanol group. Preferred compounds with an alkoxysilane group are compounds selected from the group consisting of 2-(3,4 epoxycyclohexyl) ethyltrimethoxysilane, 2-(3,4 epoxycyclohexyl) ethyltriethoxysilane, (3-glycidoxypropyl)trimethoxysilane, (3-glycidoxypropyl)methyldimethoxysilane, (3-glycidoxypropyl)trimethoxysilane, (3-glycidoxypropyl) methyldiethoxysilane, (3-glycidoxypropyl)dimethylethoxysilane, (3-glycidoxypropyl)triethoxysilane, 5,6-epoxyhexyltriethoxysilane, 2-(3,4-epoxycyclohexyl)ethylmethyldiethoxysilane, 1-(3-glycidoxypropyl)-1,1,3,3,3-pentaethoxy-1,3-disilapropane and 8-glycidoxyoctyltrimethoxysilane.

Preferably the (neutralized) compatibilizer is dispersed as a nanodispersion in water. It is hereby understood that a nanodispersion is a dispersion wherein the droplets of compatibilizer in the continuous phase are of a nanometer scale, i.e., the D90 of the droplets of the compatibilizer in liquid medium is at most 350 nm, preferably at most 250 nm, more preferably at most 100 nm, even more preferably at most 50 nm, and even more preferably at most 10 nm as measured using dynamic light scattering (e.g., using a Malvern and ASTM E3247). In one embodiment, the average particle size (z-average) of the neutralized compatibilizer in the liquid medium is at most 350 nm, preferably at most 250 nm, more preferably at most 100 nm, even more preferably at most 50 nm.

It is furthermore observed that the presence of the tertiary amine successfully facilitates the dispersion of the compatibilizer. It is speculated that this process works by transforming part of the compatibilizer into a transient ionic surfactant that facilitates dispersion by preferentially locating at the water-polymer interface.

Preferably the molar ratio between the tertiary amine and the carboxylic acid moieties is between 0.4 and 2.0, preferably between 0.5 and 1.0, more preferably between 0.6 and 0.8.

The compatibilizer is preferably selected from the group consisting of polylactides (PLA), poly-L-lactide (PLLA), poly-DL-lactide (PDLA), L-lactide/DL-lactide copolymers (PLDLA), polyglycolide (PGA), poly($\epsilon$-caprolactone) (PCL), copolymers of glycolide, copolymers of $\epsilon$-caprolactone, copolymers of lactide, glycolide/trimethylene carbonate copolymers (PGA/TMC), lactide/tetramethylglycolide copolymers, lactide/trimethylene carbonate copolymers, lactide/$\delta$-valerolactone copolymers, lactide/$\epsilon$-caprolactone copolymers, glycolide/lactide copolymers (PGLA), lactide/glycolide/trimethylene carbonate terpolymers, lactide/glycolide/$\epsilon$-caprolactone terpolymers, PLA/polyethylene oxide copolymers, unsymmetrically 3,6-substituted poly-1,4-dioxane-2,5-diones, polyhydroxybutyrates (PHB), PHB/$\beta$-hydroxyvalerate copolymers (PHB/PHV), poly-$\beta$-hydroxypropionate (PHPA), poly-p-dioxanone (PPD), poly-$\delta$-valerolactone-poly-$\epsilon$-caprolactone, poly($\epsilon$-caprolactone-DL-lactide) copolymers, polyesters of oxalic acid, poly-$\beta$-malic acid (PMLA), poly-$\beta$-alkanoic acids, and mixtures thereof. Additionally, or alternatively, the compatibilizer has a number average molecular weight ($M_n$) of at most 50 kg per mole, preferably at most 35 kg per mole, more preferably at most 30 kg per mole and even more preferably at most 25 kg per mole. The compatibilizer preferably has a molecular weight of at least 2.0 kg per mole, in particular at least 3.0 kg per mole. The suitable weight of the compatibilizer in an individual case will also depend on the nature of the compatibilizer. The molecular weights of these polymers were measured via a relative GPC analysis using polystyrene calibration as described in detail in the examples. The compatibilizer is preferably selected from the group of polylactide, poly(lactide-co-glycolide), poly(lactide-co- $\epsilon$-caprolactone), polyglycolide (PGA), and poly($\epsilon$-caprolactone) (PCL). Further preferred compatibilizers include hydrophilic polyester copolymers based on polyethylene glycol monomers, in particular copolymers of polyethylene glycol with lactide, glycolide, and/or caprolactone.

The tertiary amine is preferably selected from the group consisting of trimethylamine, triethylamine, tripropylamine, tributylamine, ethyldimethylamine, methyldiethylamine, dimethylethanolamine, diethylethanolamine, N'-methyl morpholine, N'-ethyl morpholine, pyridine, 2-methyl pyridine, 3-methyl pyridine, 4-methyl pyridine and, 2,6-lutidine.

Preferably, at least part of the tertiary amine is removed at elevated temperature (80-140 °C) under reduced pressure (i.e., lower than 100 kPa, preferably lower than 50 kPa).

Preferably the compatibilizer is in the molten state or dissolved in an organic solvent, when combined with the tertiary amine to form the dispersion of the neutralized compatibilizer in a liquid medium.

It may be preferred that the compatibilizer is provided with the carboxylic acid moieties by converting an alcohol moiety of the thermoplastic, resorbable and biocompatible polymer into a carboxylic acid moiety by ring-opening reaction with an anhydride and/or synthesizing the polymer from a suitable cyclic ester monomer (or suitable mixture of a plurality of such monomers) and an $\alpha$-hydroxy acid initiator. Alternatively, the compatibilizer with acid groups may be prepared by polycondensation of diacids and diols using a slight excess of acid according to methods described in the field of polymer chemistry.

Preferably the molar ratio of the coupling agent to the amount of carboxylic acid moieties is at least 0.5. In other words, preferably at least two carboxylic acid moieties are provided for each silane moiety. A larger number of carboxylic acid moieties increases the chance that the carboxylic acid moieties reach and chemically bond to the glass fiber.

The invention further relates to a kit of parts for coating a resorbable, biocompatible glass fiber comprising:

- a compatibilizer, comprising an amount of a thermoplastic, resorbable and biocompatible polymer comprising carboxylic acid moieties,
- a volatile tertiary amine, an aqueous liquid medium, and
- a coupling agent with at least one epoxy moiety and at least one silane moiety.

Preferably the kit is a medical kit, whereby it is understood that the kit is intended and suitable for manufacturing a medical device.

Preferably the kit further comprises a resorbable, biocompatible and preferably bioactive glass fiber. The kit of parts may further comprise instructions for carrying out the method according to the invention.

As indicated above, the present invention also pertains to a composite comprising coated glass fibers of the present invention and a resorbable and biocompatible polymer matrix. Examples of suitable resorbable and biocompatible polymer matrix materials include polymers selected from the group of polylactides (PLA), poly-L-lactide (PLLA), poly-DL-lactide (PDLA), L-lactide/DL-lactide copolymers (PLDLA), polyglycolide (PGA), poly($\epsilon$-caprolactone) (PCL), copolymers of glycolide, copolymers of $\epsilon$-caprolactone, copolymers of lactide, glycolide/trimethylene carbonate copolymers (PGA/TMC), lactide/tetramethylglycolide copolymers, lactide/trimethylene carbonate copolymers, lactide/$\delta$-valerolactone copolymers, lactide/$\epsilon$-caprolactone copolymers, glycolide/lactide copolymers (PGLA), lactide/glycolide/trimethylene carbonate terpolymers, lactide/glycolide/$\epsilon$-caprolactone terpolymers, PLA/polyethylene oxide copolymers, unsymmetrically 3,6-substituted poly-1,4-dioxane-2,5-diones, polyhydroxybutyrates (PHB), PHB/$\beta$-hydroxyvalerate copolymers (PHB/PHV), poly-$\beta$-hydroxypropionate (PHPA), poly-p-dioxanone (PDO), poly-$\delta$-valerolactone-poly-$\epsilon$-caprolactone, poly($\epsilon$-caprolactone-DL-lactide) copolymers, polyesters of oxalic acid, poly-$\beta$-malic acid (PMLA), poly-$\beta$-alkanoic acids, and mixtures thereof. As for the compatibilizer, the polymer matrix is preferably selected from the group of polylactide, poly(lactide-co-glycolide), poly(lactide-co- ε-caprolactone), polyglycolide (PGA), and poly(ε-caprolactone) (PCL).

In one embodiment, the compatibilizer and the polymer matrix are built up from the same types of monomers, more in particular in the same ratio. This will help to ensure good compatibility between the coated glass fiber and the matrix.

In one embodiment, I at least 10% of the structural units of the compatibilizer are identical to the structural units of the matrix polymer. This is one way to ensure good compatibility of the glass fiber with the matrix material. In one embodiment, at least 20% of the structural units of the compatibilizer are identical to the structural units of the matrix polymer, or at least 40%, or at least 60%. In one embodiment, both the compatibilizer and the matrix polymer comprise at least 20 wt.% of lactide units, in particular at least 30 wt.%. In one embodiment, both the compatibilizer and the matrix polymer comprise at least 20 wt.% of caprolactone units, in particular at least 30 wt.%. In another embodiment, the compatibilizer and the matrix polymer comprise at least 20 wt.% of glycolide units, in particular at least 30 wt.%.

Preferably the inherent viscosity of the matrix polymer in the composite material is between 1.5 and 4.0 dL/g, preferably between 1.8 and 3.0 dL/g, more preferably between 2.0 and 3.0 dL/g.

In general, the polymer composite material has a Young's modulus (tested per ASTM D7264/D7264M) which is higher than the Young's modulus of the matrix polymer, preferably between 110% and 1000% compared to the matrix polymer, more preferably between 200% and 600%, most preferably between 300% and 500%.

Preferably the glass reinforced polymer composite material has a mechanical strength according to a three-point bend test (tested per ASTM D7264/D7264M) higher than the strength of the matrix polymer, preferably at least 110% compared to the matrix polymer, preferably at least 200% compared to the matrix polymer, most preferably at least 400% compared to the matrix polymer when the applied force is perpendicular to the reinforcement fibers.

Preferably, the composite has a glass fiber content, calculated on the total of glass and polymer of between 5 and 95 wt.%, preferably between 10 and 90 wt.%, more preferably between 15 and 80 wt.%, even more preferably between 20 and 70 wt.%, most preferably between 30 and 60 wt.%. Alternatively, the composite preferably has a glass fiber content of at least 60 wt.%, more preferably at least 70 wt.%, even more preferably at least 80 wt.% and even more preferably at least 90 wt.%.

The composite according to the invention may comprise further components in addition to glass fiber and polymer, e.g., up to 30 wt.%, up to 20 wt.%, or up to 10 wt.%. Examples of further components may be hydroxy-apatite and calcium phosphate, such as tricalciumphosphates (TCP), e.g. β-TCP. It may be preferred for the composite to comprise an additive, for instance an active pharmaceutical ingredient (API) or a mineral ingredient, embedded within the composite.

The invention also pertains to a medical device comprising coated glass fibers according to the invention or the composite according to the invention in a polymer matrix as described above. An attractive composite and method for its manufacture is described in a patent application with the same applicant, inventors, and filing date as the present application, with the title "Biocompatible and resorbable polymer composite material and method for obtaining such".

As the skilled person is aware, glass fibers are generally manufactured in the form of fiber bundles, wherein a plurality of individual fibers, also often indicated as filaments, are obtained as a bundle from a glass manufacturing process. The fiber bundle is sometimes also indicated as yarn or strand. The present invention relates to coated glass fibers, and to a method for coating the glass fibers. This coating procedure may often be carried on the glass fibers in the bundle, but by proper spreading of the fibers it will be ensured that the individual fibers are provided with the coating. The coated glass fibers may be incorporated into a composite. In the composite, the glass fibers or glass fiber bundles may be present as continuous fibers and/or a chopped fibers. Chopped fibers, if used, generally have a length in the range of 1 mm to 50 mm, in particular 1 mm to 25 mm, more in particular 1 mm to 20 mm, even more in particular 2 mm to 10 mm, most in particular below 4mm and above 1 mm.

In one embodiment, the medical device comprises an unidirectional composite tape. Glassfiber-based tapes are known in the art. They comprise a biodegradable polymer and a plurality of unidirectionally aligned continuous glass fibers, with the fibers being aligned in the length direction of a tape. The tape generally has a width which is at least 2 times the thickness of the tapes, in particular at least 5 times, more in particular at least 10 times. The length of the tape generally is at least 10 times the width of the tape, in particular at least 100 times. The thickness of the tape is preferably less than 0.3 mm, more preferably less than 0.2 mm and even more preferably less than 0.15 mm. The length of the tape is preferably at least 1 m, more preferably at least 5 m, and even more preferably at least 10 m. The third dimension (i.e. the width) is preferably between 0.5 and 10 cm, more preferably between 0.8 and 5 cm, and even more preferably between 1 and 2.5 cm. Composite tapes can be obtained, e.g., by spreading the fibers of a glass fiber bundle, contacting the glass fibers with a polymer matrix in the liquid phase, and solidifying the matrix. Unidirectional composite tapes preferably have a matrix content of 2-40 wt.%, in particular 5-30 wt.%, more in particular 5-25 wt.%, in specific embodiments 10-20 wt.%. The preferences given elsewhere for the nature of the matrix and the nature of the glass fiber also apply here.

In one embodiment, the medical device comprises a plurality of layers, wherein one or more layers comprise one or more composite tapes.

Thus, in one embodiment, the composite is shaped in the form of an unidirectional composite tape, as discussed above. In an alternative embodiment, the composite material is shaped as a strand, (cannulated) rod, tube, pellet, or granule. A strand/rod is a round-shaped fiber reinforced polymer with a diameter that is preferably between 5 - 50 mm. Pellets are small length-wise sections of a rod having a diameter of about 5 - 50 mm and granules are small particles having a diameter of about 1 - 10 mm. The dimensions of the pellets or granules may be adapted to match the dimensions of feeding screws used in polymer processing techniques, for example injection molding.

As will be evident to the skilled person, different embodiments of the present invention can be combined unless they are mutually exclusive. The preferences on the properties of the glass fiber and the various components of the sizing also apply to the kit of parts, to the composite, and to the medical device.

All percentages used herein are weight percentages, unless specified otherwise.

The invention will be elucidated with reference to the following examples, without being limited thereto or thereby.

## Examples

## Analytical techniques

Acid number (AN):

The acid number (AN), which is the amount in mg of potassium hydroxide (KOH) required to neutralize the free acid in 1 g of compatibilizer, was determined via titration using a Metrohm 876 Dosimat Plus equipped with a Metrohm 801 stirrer. Samples were prepared by weighing 0.5 g of polymer in a 250 mL Erlenmeyer flask, followed by the addition of 100 mL pre-neutralized DCM/methanol (4/1 vol.%) mixture. After complete dissolution, three drops of 1 wt.% phenolphthalein solution in ethanol were added, and the mixture was titrated with 0.1 M ethanolic KOH until a pink color, which persisted for at least 15 seconds, was obtained. The required volume of KOH in mL (a) was noted and the acid value was calculated according to the following equation.

$$AN = a(mL) \times 56.11 \text{ g/mol} \times 0.1 \text{ M} / \text{weight sample (g)}$$

Relative gel permeation chromatography (GPC):

A gel permeation chromatographer (Agilent 1200 Series) was used to measure the relative number-averaged and weight-averaged molecular weight ($M_n$ and $M_w$, respectively) of the compatibilizer against polystyrene standards. The GPC consisted of a guard column and 2 PL gel mixed D columns and an evaporative light scattering detector (ELSD). HPLC Grade chloroform stabilized with ethanol (Biosolve) was used as the mobile phase at a flow rate of 1 mL/min and the measurement was conducted at 35 °C. Samples of 15 mg of compatibilizer were dissolved in 15 mL of chloroform and then filtered through 0.45 $\mu$m filter prior to GPC analysis. The GPC system was calibrated using narrow polystyrene standards.

Headspace GC-MS method for TEA quantification:

100 mg of the coated glass fibers were extracted in dimethyl sulfoxide (DMSO) containing deuterated triethylamine (d-TEA) as internal standard. Imidazole was added to the DMSO to release the TEA from the compatibilizer. The samples were incubated at 100 °C for twenty minutes before a portion was analyzed using a headspace GC-MS apparatus. The column used was TG-624 (30 m x 0.25 mm x 1.40 $\mu$m) and helium was used as carrier gas.

A calibration curve for TEA was prepared using the same internal standard as the samples. The results were normalized with the polymer content present on glass fiber as measured by loss on ignition technique. The GC-MS quantification was done in duplicate for each sample.

Particle size of nanodispersions:

The particle size distribution was measured using dynamic light scattering using a Malvern ZetaSizer nano. The test was performed on diluted aqueous samples in disposable plastic cuvettes following the ASTM E3247 method. At least three measurements were taken for each sample and an average was reported. D90 values were determined by this method.

Water content quantification:

A coulometric Karl-Fisher titration method was used to determine moisture content on glass sized samples.

Critical parameters were the sample weight and vial temperature. Testing was conducted with a sample weight of 0.1 ± 0.01 gram and a temperature of 130 °C.

Loss on ignition method:

The loss on ignition technique (LOI) is a technique suitable to determine the sizing content on glass fiber samples. In a LECO 701 macro thermogravimetric analyzer (TGA), the sizing content on 1 ± 0.2 g of sample was determined. The test began by logging in the samples into the software followed by the determination the initial (empty) crucible weights (with lids). Once the initial weights have been taken, the samples were added to the crucibles in their respective position and the equipment would determine the weight of the samples. The instrument subsequently heats up to 110 °C which is maintained for 30 minutes. After said 30 minutes the equipment was heated to 565 °C at a rate of 25 °C/min and is held at that temp for 2 hours. The weights were determined at regular time intervals whilst the instrument was running and the % mass loss for each step is calculated and reported. The first (110 °C step) provided the moisture content in the sample without burning any sizing from fibers. The higher temperature (565 °C) provided the dry sizing content by burning off the sizing on glass fibers.

Single fiber Pull-out test method to measure Interfacial Shear Strength (IFSS)

A single fiber was separated from the bundle of fibers by soaking the bundle in acetone. One section of the same fiber was used to measure the diameter of the fiber. The other section was embedded in polymer in the TextTechno FimaBond. A granule of polymer was melted in an aluminum crucible at 270 °C for 5 minutes. While the polymer was melting a single fiber was inserted in the hollow needle located just above the crucible containing polymer. Upon complete melting of the polymer the fiber was brought down in the center of the molten polymer blob just touching the polymer. The fiber was then embedded in the molten polymer at 220 micron embedding depth. The polymer was then kept at 150 °C for another minute to allow chain entanglement and then cooled down to room temperature. The sample was removed from the FimaBond and placed upside down in the Favimat equipped with the FimaTest fiber pull-out fixture. The Favimat was equipped with a very sensitive 210cN load cell. The fiber was grabbed with rubber faced grips right next to the polymer and pulled at 0.6mm/min. The test was performed at ambient conditions. Once the force drops to zero the test stopped and the apparent interfacial shear strength (IFSS) was automatically calculated by the software based on fiber diameter, embedding depth and maximum force. At least ten samples were tested for each group of fibers and an average is reported.

Example 1: Synthesis of carboxylic acid functionalized poly(lactide/ε-caprolactone) 75/25 molar ratio (compatibilizer)

A mixture of L-lactide (3.50 mol) and ε-caprolactone (1.17 mol) was polymerized in bulk using tin 2-ethyl hexanoate as a catalyst and 1,4-butanediol (0.22 mol) as initiator. The resulting poly(lactide/ε-caprolactone) diol was acid-functionalized by subsequent reaction with succinic anhydride (0.44 mol). The residual monomers were removed by ultrafiltration. The final polymer was amorphous (Tg = 18 °C) and had a weight-average molecular weight (Mw) of 5.6 kg/mol, an number-average molecular weight (Mn) of 3.6 g/mol, both determined by GPC relative to polystyrene standards, and an acid number of 26.9 mg KOH/g polymer. The residual amount of Sn was 140 ppm, as determined via ICP.
In a similar fashion compatibilizers with different molecular weight and acid numbers were produced and the properties are summarized in Table 1.

*Table 1: Overview of compatibilizers.*

| Compatibilizer | Mn (kg/mol) | Mw (kg/mol) | AN (mg KOH/g polymer) |
|---|---|---|---|
| 1 | 3.6 | 5.6 | 26.9 |
| 2 | 5.0 | 8.0 | 31.1 |
| 3 | 20.2 | 29 | 8.9 |
| 4 | 22.3 | 33.2 | 7.6 |
| 5 | 25.2 | 43.4 | 6.5 |
| 6 | 5.0 | 8.0 | 32.5 |

Example 2: Preparing a dispersion of compatibilizer in water

A dispersion of compatibilizer in water can be prepared using the solvent assisted method or using the melt dispersion method.

*(a) Solvent assisted method*

Poly(lactide/$\varepsilon$-caprolactone) 75/25 molar ratio initiated with 1,4-butanediol and end-capped with succinic anhydride (acid number: 26.9 mg KOH/g polymer was dissolved in acetone (43 wt.%)). The copolymer was neutralized by adding triethylamine (0.6 mol TEA/mol COOH). The neutralized copolymer was added to Milli-Q water at about 20 °C and the acetone was gently removed under vacuum. A bluish dispersion (about 20 wt.%) was obtained having a particle size (D90) of 100 nm (measured via dynamic light scattering method using a ZetaSizer-nano according to ASTM E3247).

*(b) Melt dispersion method*

A dispersion of a compatibilizer in water was obtained by melting poly(lactide/$\varepsilon$-caprolactone) 75/25 molar ratio initiated with 1,4-butanediol and end-capped with succinic anhydride (acid number: 26.9 mg KOH/g polymer) at 90 °C and adding triethylamine (0.9 mol TEA/mol COOH) to said polymer. After the mixture had been mixed for 5 minutes, pre-heated Milli-Q water at 50 °C was added and stirring was continued until a blueish dispersion (about 20 wt%) was obtained having a particle size (D90) of 260 nm.

Example 3: Influence of neutralization degree

This experiment exemplifies the influence of the degree of neutralization on the final properties of the nanodispersion (aq.). 9 grams of acid functionalized, random poly(lactide/$\varepsilon$-caprolactone) with 75/25 molar ratio with an acid number of 31.1 mg KOH/g were dissolved in 200 mL of acetone. After complete dissolution, a predetermined amount of TEA was added. The solution was subsequently stirred for 30 minutes for neutralization purposes in a sealed container at ambient temperature. The solution was then gradually added to 200 mL Milli-Q water under stirring. The obtained dispersion was agitated in an open beaker for about 24 hours in a fume hood until all the acetone evaporated leaving ~5 wt.% polymer in water. The particle size was measured using a Malvern Zeta sizer nano. Table 2 summarizes the particle sizes of the obtained dispersions at different degrees of neutralization (0.4, 0.5, 0.6, 0.7 and 1 mol of TEA per mol of acid).

*Table 2: Summary of properties of obtained dispersions.*

| TEA:Acid / mol:mol | PSD (D90) (nm) |
|---|---|
| 0.4 | 249 |
| 0.5 | 207 |
| 0.6 | 127 |
| 0.7 | 167 |
| 1.0 | 114 |

Example 3a: preparing dispersion in the absence of TEA

The procedure of Example 3 was repeated, except that no TEA was added to the polymer solution. As in Example 3, the polymer solution was gradually added to 200 mL Milli-Q water under stirring. The obtained dispersion was agitated in an open beaker for about 24 hours in a fume hood until all the acetone evaporated leaving ~5 wt.% polymer in water. The polymer was not in the form of a nano-dispersion. Rather, large particles were formed, which agglomerated and sedimented to the bottom of the container. The PSD (D90) was estimated as being in the order of tens to hundreds of microns which could not stay suspended and hence could not be measured via DLS (dynamic light scattering) device like the ZetaSizer-nano.

Example 4: Influence of molecular weight

Acid functionalized, random poly(lactide/$\varepsilon$-caprolactone) copolymers with 75/25 molar ratio, various molecular weights and accordingly acid numbers (compatibilizers 2, 3, 4, and 5 from Example 1) were dissolved in acetone at 5 wt.%,

followed by the addition of triethylamine in 1:1 mol ratio to acid. After stirring the solution for 30 minutes the polymer solution was gradually added to purified Milli-Q water. After allowing the acetone to evaporate in a fume hood over a time span of 24hr, 5 wt.% aqueous polymer dispersions were obtained. The particle size (D90) was measured by DLS (ZetaSizer). Table 3 provides the molecular weight data, the acid number data, and the particle size data.

*Table 3: Compatibilizer Mn acid number, and D90 in aqueous nanodispersion (5 wt.%)*

| Compatibilizer | Mn (kg/mol) | Acid number (KOH/g) | PSD (D90) (nm) |
|---|---|---|---|
| 2 | 5 | 31.1 | 7.7 |
| 3 | 20.2 | 8.9 | 317 |
| 4 | 22.3 | 7.6 | 268 |
| 5 | 25.2 | 6.5 | 260 |

<u>Example 5: Sizing synthesis and testing</u>

An acid functionalized poly (lactide/ε-caprolactone) copolymer with 75/25 mol%, Mn 5 kg/mol and Mw 8 kg/mol (as measured by relative GPC), was dissolved in acetone to prepare a 10% (wt./wt.). The acid number of the copolymer was 32.5 mg KOH/g. Once completely dissolved, triethylamine (TEA) was added to the vessel at a 1:0.6 molar ratio carboxylic acid/TEA to neutralize the acid. The neutralized polymer solution was then gradually added to Milli-Q water (same amount as acetone) agitated with a mixer. The acetone was evaporated and removed by applying vacuum (-15 to -20 in Hg) for at least 7 hours or until all the acetone was removed. It was assumed that all the acetone is removed from the system when. and a solids' content of over 10% was achieved. This provided an aqueous dispersion with D90 < 350 nm and a pH below 7.8.. The dispersion was then mixed with a hydrolyzed silane (3- glycidyloxypropyltriethoxysilane) such as to achieve 0.5 mol ratio to the acid in the final sizing) solution to achieve 5 wt.% solids in the sizing.

This sizing was then applied via a kiss roller onto freshly drawn glass fibers with the target composition of $SiO_2$ 67.8 wt.%, $P_2O_5$ 1.5 wt.%, $B_2O_3$ 2.3 wt.%, CaO 9 wt.%, MgO 5.4 wt.% and $Na_2O$ 14 wt.% as they are formed in a melt spinning process. The fibers were wound onto a core for further processing.

The wet glass fiber cakes were dried for 24 hours under air flow followed by 24 hours drying under vacuum (p = 0.66 kPa). The water content was below 1000 ppm and the TEA content was 3448 ppm, which corresponds to a TEA/COOH mol ratio of 0.05 or 1:20. In the drying step the reaction of silanol groups with the glass was facilitated.

The fiber cakes were then cured at 90 °C for 16 hours to promote the reaction between the acid end group of the compatibilizer and the epoxy groups. After curing, the cakes were subjected to deep vacuum for 2 hours at 90°C for removal of the remaining TEA. The sizing content was 1.2 wt.% as determined by the loss on Ignition TGA method. The amount of TEA was quantified, and it was found to be 2 ppm on the coated glass fibers.

<u>Example 6: Grafting efficiency</u>

Depending on the composite fabrication process, varying amounts of sizing may be required. For a sizing compatible with a thermoplastic matrix polymer, it might be preferred to have at least a monolayer sizing. For a given glass diameter and specific molecular weight compatibilizer, the amount of this monolayer in terms of weight percentage of glass can be calculated. For a glass fiber filament with a diameter of 12 micron, and a compatibilizer with Mn of 3 kg/mol, the calculated amount of sizing to create a covalently bonded monolayer on glass fiber with the help of an epoxy silane is around 0.3 wt.% of glass fiber. The calculation also assumes that the silanol bonds available to react with the epoxy silane had a density of 4.6 $OH/nm^2$. This is based on literature that suggests that the average density of silanols in amorphous silica is typically ~ 4.8 $OH/nm^2$. It is an a priori assumption that the covalently bonded sizing should not wash off with acetone.

To confirm the grafting of sizing on glass fibers, sized fibers obtained in accordance with the procedure described in Example 5 were soaked in acetone for 10 minutes and then washed two more times with clean acetone to remove residual unreacted compatibilizer. The samples were dried and then analyzed for sizing content via loss on ignition technique using a LECO macro TGA 701. The sizing content on the glass fibers prior to washing was 0.94 wt.% whilst the sizing content after washing off unreacted sizing from the glass fibers was around 0.39 wt.% of the glass fibers. This experiment confirmed that a monolayer of sizing was present which was covalently bonded to the glass fiber surface. The excess and unreacted sizing helps to keep the fibers together in the bundle and protects them against abrasion during fibers winding and composite fabrication processes.

Example 7: Effect of compatibilizer

The adhesion strength was directly measured using the single fiber pull-out method. Glass fibers were first treated with silane and cured. The treated glass fibers were subsequently provided with a coating comprising the compatibilizer used in Example 5, followed by a curing step to react the compatibilizer with the silane. A single glass fiber filament was then embedded in the matrix polymer (Purasorb PLDL 7030) and pulled out using a Fimatest fixture in Favimat machine. The silane only treated glass fiber were used as a negative control.

It was found that the apparent interfacial shear strength (IFSS) for the silane treated fibers was $36 \pm 17$ MPa which jumped to $42 \pm 15$ MPa for the fibers coated with calculated Mn = 3 kg/mol PLC compatibilizer. Further improvement was noticed for glass fibers coated with PLC compatibilizers prepared to have calculated Mn values of 13 kg/mol and 17 kg/mol , where the IFSS was measured to be $51 \pm 9$ and $53 \pm 8$ MPa, respectively.

Example 8 - Providing glass fibers with a sizing having polycaprolactone as compatibilizer, and incorporation of the coated fiber into a polycaprolactone matrix to form a polycaprolactone glass fiber-reinforced composite material, and further processing thereof to form a composite plaque

*1. Preparation of Silane treated glass fibers*

Hydrolyzed silane solution was prepared by dissolving 117g of 3-glycidyloxy¬propyltriethoxysilane in 10kg of ASTM type II purified water at 50 degrees Celsius for 90 minutes. Hydrolyzed silane solution was then applied in-line via a kiss roller onto freshly drawn glass fibers as they are formed in a melt spinning process. The composition of the glass corresponded to that applied in Example 5. The resulting fiber bundle was wound onto a core for further processing. The wet glass fibers were air-dried and then vacuum-dried at room temperature to achieve moisture level under 1000ppm. The dried glass fibers were heat treated at 90°C for 4 hours to covalently bond silane with hydroxyl groups on the glass fiber surface.

*2. Preparation of aqueous PC (IV 0.15) nanodispersion*

40g of acid-functionalized polycaprolactone with an IV of 0.15 dL/g (measured according to ASTM 2857) and acid number of 39.4 mg KOH/g were dissolved in 1600g or acetone for 1 hour. After complete dissolution, triethylamine (TEA, from Sigma Aldrich), in 1:1 mol ratio to acid, was added to neutralize the acid end groups. After stirring for 15 minutes, the polymer solution was gradually added to ASTM type II purified water. After removing the acetone through evaporation for 24hr, an aqueous nanodispersion was obtained with a solids content of about 3 wt.%. The average particle size (z-average) as measured by DLS (Malvern ZetaSizer-nano) was found to be 37 nanometers, D90 was 39nm.

*3. Preparation of PC (IV 0.15) sized glass fibers*

The silane treated glass fibers (with an average diameter of 16 micron) obtained in step 1 above were further treated with compatibilizer polymer (PC, IV 0.15 dL/g) prepared in step 2 above by passing the bundle of fibers through the polymer nanodispersion bath in an off-line, reel-to-reel sizing applicator system in a continuous fashion. The line speed was 5m/min. The sized fibers were collected on a separate perforated stainless steel (SS) core. The fibers were subsequently vacuum dried at room temperature and then cured at 90°C for 16 hours to facilitate reaction between the acid end groups of compatibilizer and the epoxy groups of silane already covalently bonded to the glass surface. TEA present at the reaction site acted as a catalyst that was eventually removed by applying a vacuum.

Once cured, the PC-sized glass fiber sample was tested for presence of the PC (IV 0.15) compatibilizer sizing layer via TGA loss-on-ignition method. The results confirmed the presence of $1 \pm 0.5$ wt.% sizing on glass fibers.

*4. Manufacture of a composite tape comprising polycaprolactone polymer and fibers sized with polycaprolactone compatibilizer*

10 wt.% solution of high molecular weight polycaprolactone (Purasorb PC17, IV 1.7 dL/g, commercially available from Corbion Purac) was prepared in acetone at 50°C. One single bundle (576 filaments) of the PC-sized fibers obtained in step 3 was coated with the polymer by passing it through the polymer solution bath in a continuous reel-to-reel applicator system. The polymer solution bath was equipped with rollers and pins to spread fibers for better wet-out. The solvent was removed by passing the coated fibers through an anti-solvent (ethyl alcohol) bath at room temperature at a line speed of 2m/min, followed by air drying for 24 hours. After drying, the polymer-coated fibers appeared as thin and narrow tape. The coated and dried fibers were analyzed for fiber/polymer content via LECO, TGA701 loss on ignition method. The polymer content was 14.2 wt.%.

*5. Composite plaques obtained by compression molding of the composite comprising polycaprolactone and coated glass fibers*

The composite tape obtained under step 4 was cut to 100mm long strips, and a predetermined amount was stacked and spread uniformly in the cavity of a preheated mold in a uniform direction to make samples for mechanical testing. Additional polymer (Purasorb PC17, Tm 60°C) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

*6. Mechanical testing of the composite*

The composite plaque from step 5 above was cut into rectangular coupons (50x13x1 mm; LxWxT) and tested for flexural properties (3-point-bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of $147 \pm 7.6$ MPa (a 600% improvement from base polymer strength of 23MPa) and modulus of $6.46 \pm 0.68$ GPa (58x improvement from 110MPa of base polymer) was measured for 6 samples tested. The tested samples were analyzed for fiber content via TGA loss on ignition method and the average fiber contents were found to be 50.4 wt.% of the composite.

Example 9 - Providing glass fiber having poly(DL lactide) (PDL) as compatibilizer, and incorporation of the coated fiber into a poly(DL lactide) matrix to form a poly(DL lactide) glass fiber-reinforced composite material, and further processing thereof to form a composite plaque

Epoxy-functional, silane-treated glass fiber was prepared as described in Example 8 above.

30g of acid functionalized poly(DL lactide)(50/50) polymer with an IV of 0.4 dL/g and acid number of 4.1 mg KOH/g were dissolved in 1500g acetone for 1 hour. After complete dissolution, TEA, in 1:1.3 mol ratio to acid, was added to neutralize the acid end groups. After stirring the solution for 15 minutes the polymer solution was gradually added to purified ASTM type II purified water. After removing the acetone through evaporation for 24hr a ~3 wt.% aqueous dispersion was obtained. The average particle size (z-average) as measured by DLS (Malvern ZetaSizer-nano) was found to be 30 nanometers, D90 was 24nm.

The silane-treated glass fibers were further treated with compatibilizer polymer by passing the bundle of fibers through a bath of the polymer nanodispersion described above in an off-line reel-to-reel sizing applicator system in a continuous fashion. The line speed was 5m/min. The sized fibers were collected on a separate perforated SS core. The fibers were vacuum dried at room temperature and then cured at 90°C for 16 hours to facilitate reaction between -COOH end group of compatibilizer polymer and epoxy end of silane already covalently bonded to glass surface. TEA present at the reaction site acted as a catalyst which was eventually removed by applying a vacuum.

Once cured, the glass sample was tested for presence of sizing layer via TGA loss-on-ignition method. The results confirmed presence of $1 \pm 0.5$ wt.% sizing on glass fibers.

10 wt.% solution of high molecular weight poly(DL lactide) (IV 2.0 dL/g) was prepared in acetone. One single bundle (576 filaments) of the sized fibers described above (with an average diameter of 16 micron) was coated with high molecular weight poly(DL lactide) by passing it through the polymer solution bath in a continuous reel-to-reel applicator system. The polymer solution bath was equipped with rollers and pins to spread fibers for better wet-out The solvent was removed by passing the coated fibers through an anti-solvent (ethylalcohol) bath at room temperature. The line speed was 1 m/min. The coated fibers were air dried for 24 hours. After drying the coated fibers appeared as thin and narrow tape. The coated and dried fibers were analyzed for fiber/polymer content via LECO TGA701 loss on ignition method. The polymer content was 15.4 wt.%.

The composite tape obtained was cut to 100mm long strips, and a predetermined amount was stacked and spread uniformly in the cavity of a preheated mold in a uniform direction to make samples for mechanical testing. Additional polymer (Purasorb PLDL7020) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

The composite plaque was then cut into rectangular coupons (50x13x1 mm; LxWxT) and tested for flexural properties (3-point bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of $491.3 \pm 38.6$ MPa (a 446% improvement from base polymer strength of 110MPa) and modulus of $12.72 \pm 1.5$ GPa (374% improvement from 3.4 GPa of base polymer) was measured for 6 samples tested. The tested samples were analyzed for fiber content via TGA loss on ignition method and the average fiber contents were found to be 52 wt.% of the composite.

Example 10 - Providing glass fiber with a sizing having poly(DL lactide/glycolide) (PDLG) as compatibilizer, and incorporation of the sized fiber into a poly(DL lactide/glycolide) matrix to form a poly(DL lactide/glycolide) glass fiber-reinforced composite material, and further processing thereof to form a composite plaque

Epoxy-functional, silane-treated glass fiber was prepared as described in Example 8 above.

30g of acid functionalized poly(DL lactide/glycolide) 50/50 from Corbion with an IV of 0.4 and acid number of 4.1 mg KOH/g was dissolved in 1500g acetone in 1 hour. After complete dissolution, triethylamine, in 1:1.3 mol ratio to acid, was added to neutralize the acid end groups. After stirring the solution for 15 minutes the polymer solution was gradually added to purified ASTM type II purified water. After removing the acetone through evaporation for 24hr a ~3 wt. % aqueous dispersion was obtained. The average particle size (z-average) as measured by DLS (Malvern ZetaSizer-nano) was found to be 46 nanometers, D90 was 46nm.

The silane treated glass fibers were further treated with the dispersion as described above by passing the glass fibers through a bath of the polymer nanodispersion in an off-line reel-to-reel sizing applicator system in a continuous fashion. The line speed was 5m/min. The sized fibers were collected on a separate perforated SS core. The fibers were vacuum dried at room temperature and then cured at 90°C for 16 hours to facilitate reaction between -COOH end group of compatibilizer polymer and epoxy end of silane already covalently bonded to glass surface. TEA present at the reaction site acted as a catalyst which was eventually removed by applying a vacuum.

Once cured, the glass sample was tested for presence of sizing layer via TGA loss-on-ignition method. The results confirmed the presence of $1\pm0.5$ wt.% coating on glass fibers.

10 wt.% solution of high molecular weight poly(DL lactide/glycolide) 85/15 with an IV of 2.3 dL/g was prepared in acetone at 50°C. One single bundle (576 filaments) of the PDLG sized fibers described above (with an average diameter of 16 micron) was coated with PDLG85/15 by passing it through the polymer solution bath in a continuous reel to reel applicator system. The polymer solution bath was equipped with rollers and pins to spread fibers for better wet-out The solvent was removed by passing the coated fibers through an anti-solvent (alcohol) bath at room temperature. The line speed was 2m/min. The coated fibers were air dried for 24 hours. After drying the coated fibers appeared as a thin and narrow tape. The coated and dried fibers were analyzed for fiber/polymer content via LECO TGA701 loss on ignition method. The polymer content were 32.6 wt.%.

The composite tape obtained was cut to 100mm long strips, and a predetermined amount was stacked and spread uniformly in the cavity of a preheated mold in a uniform direction to make samples for mechanical testing. Additional polymer (Purasorb PLDG85/15) film was added as a top layer on the coated fiber stack to make up 50 wt.% fibers in the final molded part. The cavity of the mold was 100mmx100mmx1mm. These coated fiber bundles and polymer film layers were then heated above the softening temperature of the polymer, pressed and subsequently cooled and removed from the mold. A composite plaque was obtained, which was used for analytical purposes.

The composite plaque was cut into rectangular coupons (target 50x13x1 mm ; LxWxT) and tested for flexural properties (3pt bending test) according to ASTM D7264/D7264M-15 using a Lloyd universal mechanical tester equipped with 3 point bend fixture and 2.5kN loadcell. Span to thickness: 16:1 ratio was used. Standard width: 13mm with the specimen length being about 20% longer than the support span (10% each side). An average strength of $770.7\pm131$ MPa (a 770% improvement from base polymer strength of 100MPa) and modulus of $23.9\pm5.4$ GPa (570% improvement from 4.2 GPa of base polymer) was measured for 6 samples tested. The tested samples were analyzed for fiber content via TGA loss on ignition method and the average fiber contents were found to be 55.7 wt.% of the composite.

**Claims**

**1.** Resorbable, biocompatible glass fiber, coated with a sizing wherein the sizing comprises a thermoplastic, resorbable and biocompatible compatibilizer that is covalently bonded to the glass fiber through a coupling agent having at least one silane moiety, wherein the glass fiber has an apparent interfacial shear strength with a reference matrix polymer as determined in the single fiber pullout test which is at least 10% higher than the apparent interfacial shear of a bare glass fiber with the same reference matrix polymer, wherein the thermoplastic, resorbable and biocompatible compatibilizer that is covalently bonded to the glass fiber through a coupling agent having at least one silane moiety is the reaction product of a coupling agent having at least one epoxy moiety and at least one silanol group and the product of the reaction of a compatibilizer which is a thermoplastic, resorbable and biocompatible polymer comprising carboxylic acid end-groups with a volatile tertiary amine in an aqueous liquid medium.

**2.** Coated glass fiber according to claim 1, wherein the coated glass fiber comprises an amount of volatile tertiary amine of less than 10 ppm, or between 0.1 ppm and 10 ppm.

3. Coated glass fiber according to claim any one of the preceding claims, wherein the sizing is free of surfactant and preferably wherein the coated glass fiber is free of surfactant.

4. Coated glass fiber according to any one of the preceding claims, wherein the coated glass fiber has an apparent interfacial shear strength with a reference matrix polymer as determined in the single fiber pullout test which is at least 20% higher than the apparent interfacial shear of a bare glass fiber with the same reference matrix polymer, in particular at least 30%.

5. Coated glass fiber according to any one of the preceding claims, wherein the coated glass fiber has an apparent interfacial shear strength with a reference matrix polymer as determined in the single fiber pullout test of at least 10 MPa, in particular at least 15 MPa, more in particular at least 20 MPa, more in particular is at least 30 MPa, preferably at least 40 MPa, and even more preferably at least 50 MPa..

6. Method for manufacturing a resorbable, biocompatible glass fiber coated with a sizing, in particular a coated resorbable and biocompatible glass fiber according to any one of claims 1-5, comprising the steps of:

   a) coating a glass fiber with a sizing composition by contacting the glass fiber with

      - a coupling agent with at least one epoxy moiety and at least one silanol group in a liquid medium and
      - the reaction product of the reaction of a compatibilizer with a volatile tertiary amine in an aqueous liquid medium, in which the compatibilizer is a thermoplastic, resorbable and biocompatible polymer comprising carboxylic acid end-groups,

   b) subjecting the coated glass fiber to a drying step,
   c) subjecting the dried coated glass fiber to a curing step at elevated temperature,
   d) subjecting the cured coated glass fiber to a step to evaporate the volatile tertiary amine.

7. Method according to claim 6, wherein the tertiary amine acts as a catalyst for the reaction between the epoxy moiety and the neutralized compatibilizer.

8. Method according to claim 6 or 7, wherein the coupling agent and the reaction product of the reaction of the compatibilizer with the volatile tertiary amine are present in the same liquid medium.

9. Method according to claim 6 or 7, wherein the coupling agent and the reaction product of the reaction of the compatibilizer with the volatile tertiary amine are present in the different liquid media, and wherein the glass fiber is first contacted with the coupling agent in a liquid medium, and then contacted with the reaction product of the reaction of the compatibilizer with the volatile tertiary amine in a liquid medium, optionally with a drying and/or curing step between the contacting with the coupling agent and the contacting with the product of the reaction of a compatibilizer with a volatile tertiary amine.

10. Method according to any one of claims 6-9, wherein the product of the reaction of a compatibilizer with a volatile tertiary amine is dispersed in the aqueous liquid medium in the form of a microdispersion, preferably in the form of a nano-dispersion.

11. Method according to any one of claims 6-10, wherein the volatile tertiary amine is selected from the group consisting of trimethylamine, triethylamine, tripropylamine, tributylamine, ethyldimethylamine, methyldiethylamine, dimethylethanolamine, diethylethanolamine, N'-methyl morpholine, N'-ethyl morpholine, pyridine, 2-methyl pyridine, 3-methyl pyridine, 4-methyl pyridine and 2,6-lutidine.

12. Method according to any one of claims 6-11, wherein at least part of the tertiary amine is removed under reduced pressure.

13. Method according to any one of claims 6-12 or glass fiber according to any one of claims 1-5, wherein the compatibilizer comprises polymer selected from the group consisting of polylactides (PLA), poly-L-lactide (PLLA), poly-DL-lactide (PDLLA), polyglycolide (PGA), poly($\varepsilon$-caprolactone) (PCL), copolymers of glycolide, glycolide/trimethylene carbonate copolymers (PGA/TMC), lactide/tetramethylglycolide copolymers, lactide/trimethylene carbonate copolymers, lactide/d-valerolactone copolymers, lactide/ε-caprolactone copolymers, L-lactide/DL-lactide copolymers (PLDLA), glycolide/L-lactide copolymers (PGA/PLLA), polylactide-co-glycolide, lactide/glycolide/trimethylene carbonate terpolymers, lactide/glycolide/ε-caprolactone terpolymers, PLA/polyethylene oxide copolymers, unsymmetrically 3,6-substituted poly-1,4-dioxane-2,5-diones, polyhydroxybutyrates (PHB), PHB/b-hydroxyvalerate copolymers (PHB/PHV), poly-β-hydroxypropionate (PHPA), poly-p-dioxanone (PDO), poly-d-valerolactone-poly-ε-caprolactone, poly(ε-caprolactone-DL-lactide) copolymers, polyesters of oxalic acid, poly-β-malic acid (PMLA), poly-β-alkanoic acids, polyorthoesters, poly(ester anhydrides), and mixtures thereof, and in particular from the group of polylactide, poly(lactide-co-glycolide), poly(lactide-co- ε-caprolactone), polyglycolide (PGA), and poly(ε-caprolactone) (PCL).

14. Kit of parts suitable for coating a resorbable, biocompatible and preferably bioactive glass fiber, comprising:

- a compatibilizer, comprising an amount of a thermoplastic, resorbable and biocompatible polymer comprising carboxylic acid moieties,
- a tertiary amine,
- an aqueous liquid medium, and
- a coupling agent with at least one epoxy moiety and at least one silane moiety

15. Composite comprising a plurality of glass fibers according to claim 1-5, or the product of the method of any one of claims 6-13 embedded in a resorbable and biocompatible polymer matrix.

16. Composite according to claim 15, wherein the composite in the form of an unidirectional composite tape, or in the form of a strand, (cannulated) rod, tube, pellet, or granule, in particular in the form of an unidirectional tape.

17. Medical device, comprising a plurality of glass fibers according to claim 1-5, or the product of the method of any one of claims 6-13, or the composite of claims 15 or 16.

## Patentansprüche

1. Resorbierbare, biokompatible Glasfaser, welche mit einer Schlichte beschichtet ist, wobei die Schlichte ein thermoplastisches, resorbierbares und biokompatibles Kompatibilisierungsmittel umfasst, welches kovalent an die Glasfaser über ein Kupplungsmittel mit mindestens einer Silaneinheit gebunden ist, wobei die Glasfaser eine scheinbare Grenzflächen-Scherfestigkeit mit einem Referenzmatrixpolymer aufweist, die in einem Einzelfaser-Auszugstest bestimmt wurde und mindestens 10% höher ist als die scheinbare Grenzflächen-Scherfestigkeit einer unbeschichteten Glasfaser mit demselben Referenzmatrixpolymer, wobei das thermoplastische, resorbierbare und biokompatible Kompatibilisierungsmittel, das über ein Kupplungsmittel mit mindestens einer Silaneinheit kovalent an die Glasfaser gebunden ist, das Reaktionsprodukt eines Kupplungsmittels mit mindestens einer Epoxideinheit und mindestens einer Silanoleinheit und des Produkts der Reaktion eines Kompatibilisierungsmittels, welches ein thermoplastisches, resorbierbares und biokompatibles Polymer ist, das Carbonsäure-Endgruppen umfasst, mit einem flüchtigen tertiären Amin in einem wässrigen flüssigen Medium ist.

2. Beschichtete Glasfaser gemäß Anspruch 1, wobei die beschichtete Glasfaser eine Menge an flüchtigem tertiärem Amin von weniger als 10 ppm oder zwischen 0,1 ppm und 10 ppm umfasst.

3. Beschichtete Glasfaser gemäß einem der vorhergehenden Ansprüche, wobei die Schlichte frei von grenzflächenaktivem Mittel ist und vorzugsweise wobei die beschichtete Glasfaser frei von grenzflächenaktivem Mittel ist.

4. Beschichtete Glasfaser gemäß einem der vorhergehenden Ansprüche, wobei die beschichtete Glasfaser eine scheinbare Grenzflächen-Scherfestigkeit mit einem Referenzmatrixpolymer aufweist, die in einem Einzelfaser-Auszugstest bestimmt wurde, welche mindestens 20% höher ist als die scheinbare Grenzflächen-Scherfestigkeit einer unbeschichteten Glasfaser mit demselben Referenzmatrixpolymer, vorzugsweise mindestens 30%.

5. Beschichtete Glasfaser gemäß einem der vorhergehenden Ansprüche, wobei die beschichtete Glasfaser eine scheinbare Grenzflächen-Scherfestigkeit mit einem Referenzmatrixpolymer, die in einem Einzelfaser-Auszugstest bestimmt wurde, von mindestens 10 MPa, insbesondere mindestens 15 MPa, besonders bevorzugt mindestens 20 MPa, besonders bevorzugt mindestens 30 MPa, vorzugsweise mindestens 40 MPa, und stärker bevorzugt mindestens 50 MPa, aufweist.

6. Verfahren zur Herstellung einer resorbierbaren, biokompatiblen Glasfaser, welche mit einer Schlichte beschichtet ist, vorzugsweise einer beschichteten, resorbierbaren und biokompatiblen Glasfaser gemäß einem der Ansprüche 1 bis 5, umfassend die Schritte:

    a) Beschichten einer Glasfaser mit einer Schlichtezusammensetzung durch Inkontaktbringen der Glasfaser mit

        - einem Kupplungsmittel mit mindestens einer Epoxideinheit und mindestens einer Silanolgruppe in einem flüssigen Medium und
        - dem Reaktionsprodukt der Reaktion eines Kompatibilisierungsmittels mit einem flüchtigen tertiären Amin in einem wässrigen flüssigen Medium, in welchem das Kompatibilisierungsmittel ein thermoplastisches, resorbierbares und biokompatibles Polymer ist, das Carbonsäure-Endgruppen umfasst,

    b) Unterziehen der beschichteten Glasfaser einem Trocknungsschritt,
    c) Unterziehen der getrockneten beschichteten Glasfaser einem Härtungsschritt bei erhöhter Temperatur,
    d) Unterziehen der gehärteten beschichteten Glasfaser einem Schritt, um das flüchtige tertiäre Amin zu verdampfen.

7. Verfahren gemäß Anspruch 6, wobei das tertiäre Amin als ein Katalysator für die Reaktion zwischen der Epoxideinheit und dem neutralisierten Kompatibilisierungsmittel wirkt.

8. Verfahren gemäß Anspruch 6 oder 7, wobei das Kupplungsmittel und das Reaktionsprodukt der Reaktion des Kompatibilisierungsmittels mit dem flüchtigen tertiären Amin in dem gleichen flüssigen Medium vorliegen.

9. Verfahren gemäß Anspruch 6 oder 7, wobei das Kupplungsmittel und das Reaktionsprodukt der Reaktion des Kompatibilisierungsmittels mit dem flüchtigen tertiären Amin in den verschiedenen flüssigen Medien vorliegen und wobei die Glasfaser zunächst mit dem Kupplungsmittel in einem flüssigen Medium in Kontakt gebracht und dann mit dem Reaktionsprodukt der Reaktion des Kompatibilisierungsmittels mit dem flüchtigen tertiären Amin in einem flüssigen Medium in Kontakt gebracht wird, gegebenenfalls mit einem Trocknungs- und/oder Härtungsschritt zwischen dem Inkontaktbringen mit dem Kupplungsmittel und dem Inkontaktbringen mit dem Reaktionsprodukt eines Kompatibilisierungsmittels mit einem flüchtigen tertiären Amin.

10. Verfahren gemäß einem der Ansprüche 6 bis 9, wobei das Reaktionsprodukt eines Kompatibilisierungsmittels mit einem flüchtigen tertiären Amin in dem wässrigen flüssigen Medium in Form einer Mikrodispersion, vorzugsweise in Form einer Nanodispersion, dispergiert ist.

11. Verfahren gemäß einem der Ansprüche 6 bis 10, wobei das flüchtige tertiäre Amin ausgewählt ist aus der Gruppe bestehend aus Trimethylamin, Triethylamin, Tripropylamin, Tributylamin, Ethyldimethylamin, Methyldiethylamin, Dimethylethanolamin, Diethylethanolamin, N'- Methylmorpholin, N'-Ethylmorpholin, Pyridin, 2-Methylpyridin, 3-Methylpyridin, 4-Methylpyridin und 2,6-Lutidin.

12. Verfahren gemäß einem der Ansprüche 6 bis 11, wobei mindestens ein Teil des tertiären Amins unter reduziertem Druck entfernt wird.

13. Verfahren gemäß einem der Ansprüche 6 bis 12 oder Glasfaser gemäß einem der Ansprüche 1 bis 5, wobei das Kompatibilisierungsmittel ein Polymer umfasst, das ausgewählt ist aus der Gruppe bestehend aus Polylactiden (PLA), Poly-L-Lactid (PLLA), Poly-DL-Lactid (PDLLA), Polyglycolid (PGA), Poly($\epsilon$-caprolacton) (PCL), Copolymeren von Glycolid, Glycolid/Trimethylencarbonat-Copolymeren (PGA/TMC), Lactid/Tetramethylglycolid-Copolymeren, Lactid/Trimethylencarbonat-Copolymeren, Lactid/d-Valerolacton-Copolymeren, Lactid/$\epsilon$-Caprolacton-Copolymeren, L-Lactid/DL-Lactid-Copolymeren (PLDLA), Glycolid/L-Lactid-Copolymeren (PGA/PLLA), Polylactid-co-Glycolid, Lactid/Glycolid/Trimethylencarbonat-Terpolymeren, Lactid/Glycolid/$\epsilon$-Caprolacton-Terpolymeren, PLA/Polyethylenoxid-Copolymeren, unsymmetrisch 3,6-substituierten Poly-1,4-Dioxan-2,5-dionen, Polyhydroxybutyraten (PHB), PHB/b-Hydroxyvalerat-Copolymeren (PHB/PHV), poly-$\beta$-Hydroxypropionat (PHPA), poly-p-Dioxanon (PDO), poly-d-Valerolacton-poly-$\epsilon$-Caprolacton, poly($\epsilon$-Caprolacton-DL-lactid)-Copolymeren, Polyestern von Oxalsäure, poly-ß-Maleinsäure (PMLA), poly-ß-Alkansäure, Polyorthoestern, poly(Esteranhydriden), und Gemischen davon, und vorzugsweise aus der Gruppe von Polylactid, poly(Lactid-co-Glycolid), poly(Lactid-co-$\epsilon$-Caprolacton), Polyglycolid (PGA), und poly($\epsilon$-Caprolacton) (PCL).

14. Kit mit Teilen, das zur Beschichtung einer resorbierbaren, biokompatiblen und vorzugsweise bioaktiven Glasfaser geeignet ist, umfassend:

- ein Kompatibilisierungsmittel, umfassend eine Menge eines thermoplastischen, resorbierbaren und biokompatiblen Polymers, das Carbonsäure-Endgruppen umfasst,
- ein tertiäres Amin,
- ein wässriges flüssiges Medium, und
- ein Kupplungsmittel mit mindestens einer Epoxideinheit und mindestens einer Silaneinheit.

15. Verbundstoff, umfassend mehrere Glasfasern gemäß Anspruch 1 bis 5, oder das Produkt des Verfahrens gemäß einem der Ansprüche 6 bis 13, eingebettet in eine resorbierbare und biokompatible Polymermatrix.

16. Verbundstoff gemäß Anspruch 15, wobei der Verbundstoff in Form eines unidirektionalen Verbundbandes oder in Form eines Strangs, eines (kanülierten) Stabs, eines Rohrs, eines Pellets oder eines Granulats, insbesondere in Form eines unidirektionalen Bandes, vorliegt.

17. Medizinische Vorrichtung, umfassend mehrere Glasfasern gemäß Anspruch 1 bis 5, oder das Produkt des Verfahrens gemäß einem der Ansprüche 6 bis 13, oder den Verbundstoff gemäß den Ansprüchen 15 oder 16.

**Revendications**

1. Fibre de verre biocompatible, résorbable, revêtue d'un encollage dans laquelle l'encollage comprend un agent de compatibilité thermoplastique, résorbable et biocompatible qui est lié de manière covalente à la fibre de verre par un agent de couplage ayant au moins une fraction silane, dans laquelle la fibre de verre présente une résistance au cisaillement interfacial apparent avec un polymère de matrice de référence telle que déterminée dans l'essai d'arrachement de fibre unique qui est au moins 10 % supérieure au cisaillement interfacial apparent d'une fibre de verre nue avec le même polymère de matrice de référence, dans laquelle l'agent de compatibilité thermoplastique, résorbable et biocompatible qui est lié par covalence à la fibre de verre par un agent de couplage ayant au moins une fraction silane est le produit réactionnel d'un agent de couplage ayant au moins une fraction époxy et au moins un groupe silanol et le produit de la réaction d'un agent de compatibilité qui est un polymère thermoplastique, résorbable et biocompatible comprenant des groupes terminaux acide carboxylique avec une amine tertiaire volatile dans un milieu liquide aqueux.

2. Fibre de verre revêtue selon la revendication 1, dans laquelle la fibre de verre revêtue comprend une quantité d'amine tertiaire volatile inférieure à 10 ppm, ou comprise entre 0,1 ppm et 10 ppm.

3. Fibre de verre revêtue selon quelconque des revendications précédentes, dans laquelle l'encollage est exempt de tensioactif et de préférence dans laquelle la fibre de verre revêtue est également exempte de tensioactif.

4. Fibre de verre revêtue selon quelconque des revendications précédentes, dans laquelle la fibre de verre revêtue présente une résistance au cisaillement interfacial apparent avec un polymère de matrice de référence telle que déterminée dans l'essai d'arrachement de fibre unique qui est au moins 20 % supérieure au cisaillement interfacial apparent d'une fibre de verre nue avec le même polymère de matrice de référence, en particulier d'au moins 30 %.

5. Fibre de verre revêtue selon quelconque des revendications précédentes, dans laquelle la fibre de verre revêtue présente une résistance au cisaillement interfacial apparent avec un polymère de matrice de référence tel que déterminé dans l'essai d'arrachement de fibre unique d'au moins 10 MPa, en particulier d'au moins 15 MPa, plus particulièrement d'au moins 20 MPa, plus particulièrement est d'au moins 30 MPa, de préférence d'au moins 40 MPa, et encore plus préférentiellement d'au moins 50 MPa.

6. Procédé pour fabriquer une fibre de verre biocompatible, résorbable revêtue d'un encollage, en particulier une fibre de verre résorbable et biocompatible revêtue selon quelconque des revendications 1 à 5, comprenant les étapes de :

a) revêtement d'une fibre de verre avec une composition d'encollage par mise en contact de la fibre de verre avec

- un agent de couplage avec au moins une fraction époxy et au moins un groupe silanol dans un milieu liquide et
- le produit réactionnel de la réaction d'un agent de compatibilité avec une amine tertiaire volatile dans un milieu liquide aqueux, dans lequel l'agent de compatibilité est un polymère thermoplastique, résorbable et biocompatible comprenant des groupes terminaux acide carboxylique,

b) soumission de la fibre de verre revêtue à une étape de séchage,
c) soumission de la fibre de verre revêtue séchée à une étape de durcissement à haute température,
d) soumission de la fibre de verre revêtue durcie à une étape pour évaporer l'amine tertiaire volatile.

7. Procédé selon la revendication 6, dans lequel l'amine tertiaire agit comme un catalyseur pour la réaction entre la fraction époxy et l'agent de compatibilité neutralisé.

8. Procédé selon la revendication 6 ou 7, dans lequel l'agent de couplage et le produit réactionnel de la réaction de l'agent de compatibilité avec l'amine tertiaire volatile sont présents dans le même milieu liquide.

9. Procédé selon la revendication 6 ou 7, dans lequel l'agent de couplage et le produit réactionnel de la réaction de l'agent de compatibilité avec l'amine tertiaire volatile sont présents dans les milieux liquides différents, et dans lequel la fibre de verre est d'abord mise en contact avec l'agent de couplage dans un milieu liquide, puis mise en contact avec le produit réactionnel de la réaction de l'agent de compatibilité avec l'amine tertiaire volatile dans un milieu liquide, éventuellement avec une étape de séchage et/ou de durcissement entre la mise en contact avec l'agent de couplage et la mise en contact avec le produit de la réaction de l'agent de compatibilité avec une amine tertiaire volatile.

10. Procédé selon quelconque des revendications 6-9, dans lequel le produit de la réaction d'un agent de compatibilité avec une amine tertiaire volatile est dispersé dans le milieu liquide aqueux sous la forme d'une microdispersion, de préférence sous la forme d'une nanodispersion.

11. Procédé selon quelconque des revendications 6 à 10, dans lequel l'amine tertiaire volatile est choisie dans le groupe consistant en triméthylamine, triéthylamine, tripropylamine, tributylamine, éthyldiméthylamine, méthyldiéthylamine, diméthyléthanolamine, diéthyléthanolamine, N'-méthyl morpholine, N'-éthyl morpholine, pyridine, 2-méthylpyridine, 3-méthylpyridine, 4-méthylpyridine et 2,6-lutidine.

12. Procédé selon quelconque des revendications 6 à 11, dans lequel au moins une partie de l'amine tertiaire est éliminée sous pression réduite.

13. Procédé selon quelconque des revendications 6 à 12 ou fibre de verre selon quelconque des revendications 1 à 5, dans lequel l'agent de compatibilité comprend un polymère choisi dans le groupe consistant en polylactides (PLA), poly-L-lactide (PLLA), poly-DL-lactide (PDLLA), polyglycolide (PGA), poly($\epsilon$-caprolactone) (PCL), copolymères de glycolide, copolymères de glycolide/carbonate de triméthylène (PGA/TMC), copolymères de lactide/tétraméthylglycolide, copolymères de lactide/carbonate de triméthylène, copolymères de lactide/d-valérolactone, copolymères de lactide/$\epsilon$-caprolactone, copolymères de L-lactide/DL-lactide (PLDLA), copolymères de glycolide/L-lactide (PGA/PLLA), polylactide-co-glycolide, terpolymères de lactide/glycolide/carbonate de triméthylène, terpolymères de lactide/glycolide/$\epsilon$-caprolactone, copolymères de PLA/oxyde de polyéthylène, poly-1,4-dioxane-2,5-diones 3,6-substitués de manière asymétrique, polyhydroxybutyrates (PHB), copolymères d'hydroxyvalérate de PHB/b (PHB/PHV), poly-$\beta$-hydroxypropionate (PHPA), poly-p-dioxanone (PDO), poly-d-valérolactone-poly-$\epsilon$-caprolactone, copolymères de poly($\epsilon$-caprolactone-DL-lactide), polyesters d'acide oxalique, acide poly-$\beta$-malique (PMLA), acides poly-$\beta$-alcanoïques, polyorthoesters, poly(anhydrides d'ester), et leurs mélanges, et en particulier le groupe des polylactide, poly(lactide-co-glycolide), poly(lactide-co-$\epsilon$-caprolactone), polyglycolide (PGA) et poly($\epsilon$-caprolactone) (PCL).

14. Kit de pièces adaptées pour le revêtement d'une fibre de verre résorbable, biocompatible et de préférence bioactive, comprenant :

- un agent de compatibilité, comprenant une quantité d'un polymère thermoplastique, résorbable et biocompatible comprenant des fractions d'acide carboxylique,
- une amine tertiaire,
- un milieu liquide aqueux, et
- un agent de couplage avec au moins une fraction époxy et au moins une fraction silane

15. Composite comprenant une pluralité de fibres de verre selon la revendication 1 à 5, ou le produit du procédé selon quelconque des revendications 6 à 13 incorporé dans une matrice polymère résorbable et biocompatible.

**16.** Composite selon la revendication 15, dans lequel le composite est sous la forme d'une bande composite unidirectionnelle, ou sous la forme d'une mèche, d'une tige (canulée), d'un tube, d'une pastille ou d'un granulé, en particulier sous la forme d'une bande unidirectionnelle.

**17.** Dispositif médical, comprenant une pluralité de fibres de verre selon les revendications 1-5, ou le produit du procédé selon quelconque des revendications 6-13, ou le composite selon les revendications 15 ou 16.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106149127 A1

**Non-patent literature cited in the description**

- **J.L. THOMASON**. *Glass fibre sizing: A review, Composites Part A*, 2019, vol. 127, 105619